# EUROPÄISCHE PATENTSCHRIFT

(11) **EP 1 716 131 B1**
(45) Veröffentlichungstag und Bekanntmachung des Hinweises auf die Patenterteilung: **29.06.2011**
(21) Anmeldenummer: 04803779.0
(22) Anmeldetag: 11.12.2004
(51) Int. Cl.: C07D 307/33, A61L 2/16

(54) **NEUE ALOXYLACTONE, ALKOXYLACTAME UND ALKOXYTIOLACTAME ZUR KONTROLLE VON AUF MIKROBIELLER INTERAKTION BERUHENDEN VORGÄNGEN**
NOVEL ALKOXYLACTONES, ALKOXYLACTAMES AND ALKOXYTHIOLACTAMES FOR CONTROLLING PROCESSES BASED ON MICROBIAL INTERACTION
NOUVELLES ALCOXYLACTONES, ALCOXYLACTAMES ET ALCOXYTHIOLACTAMES PERMETTANT DE CONTROLER DES PHENOMENES BASES SUR L'INTERACTION MICROBIENNE

(30) Priorität: 23.12.2003 DE 10361457
(43) Veröffentlichungstag der Anmeldung: 02.11.2006
(73) Patentinhaber: Henkel AG & Co. KGaA, 40589 Düsseldorf (DE)
(72) Erfinder: STUMPE, Stefan, 40593 Düsseldorf (DE); BREVES, Roland, 40822 Mettmann (DE); HUCHEL, Ursula, 50670 Köln (DE); JANSSEN, Frank, 40589 Düsseldorf (DE); HÄTZELT, André, 40591 Düsseldorf (DE)
(86) Internationale Anmeldenummer: PCT/EP2004/014141
(87) Internationale Veröffentlichungsnummer: WO 2005/063726

(56) Entgegenhaltungen:
- WO-A-02/47681
- WO-A-96/29392
- PATENT ABSTRACTS OF JAPAN Bd. 017, Nr. 119 (C-1034), 12. März 1993 (1993-03-12) & JP 04 300877 A (FUJI YAKUHIN KOUGIYOU KK), 23. Oktober 1992 (1992-10-23)
- C DAREMON, R RAMBAUD: "obtention et étude de quelques gamma-butanolides alpha-substitués (1er mémoire)" BULLETIN DE LA SOCIÉTE CHIMIQUE DE FRANCE, 1971, Seiten 294-301, XP009046464

## Beschreibung

Die vorliegende Erfindung betrifft neue Alkoxylactone, Alkoxylactame und Alkoxythiolactame sowie Verfahren zur Kontrolle von auf mikrobieller Interaktion beruhenden Vorgängen unter Einsatz dieser Alkoxylactone, Alkoxylactame und Alkoxythiolactame sowie Verwendungen dieser Alkoxylactone, Alkoxylactame und Alkoxythiolactame und Mittel, enthaltend diese Alkoxylactone, Alkoxylactame und Alkoxythiolactame.

Mikroorganismen kommunizieren miteinander über eine Vielzahl unterschiedlicher Signale. Insbesondere die Kommunikations- und Interaktionsmechanismen bei Bakterien sind im Stand der Technik ausführlich beschrieben.

In den Schriften WO 00/56154; EP 0881297; US 6287836; EP 0909820 und US 6001600 werden spezielle Histidin-Kinasen bzw. homologe Proteine dazu beschrieben, die dem Screening nach antibakteriellen Substanzen dienen sollen. Die Kinasen stammen ausschließlich aus Gram-positiven Organismen.

In der WO 01/49708 wird zum Blockieren eines Response Regulators ein Peptid mit mindestens 6 aber weniger als 200 Aminosäuren eingesetzt. Es werden jedoch keine Signalmoleküle blockiert, sondern die Aktivierung der nächsten Proteine in der Signalkette verhindert.

Die Ausbildung reifer Biofilme bei oder mit Bakterien ist von der Kommunikation zwischen bakteriellen Zellen über diverse extrazelluläre Signalstoffe abhängig.

Abhängig von der Zellzahldichte adhärierter Bakterien bewirkt die Überschreitung einer Mindestkonzentration dieser Botenstoffe eine Aktivierung der Biofilmbildung sowie eine Anschaltung weiterer Virulenzgene. Dieses Phänomen wird "Quorum Sensing" genannt und eröffnet grundsätzlich die Möglichkeit zur Kontrolle von Biofilmen, ohne die im Biofilm lebenden Keime abzutöten.

Bisher wurden zahlreiche Signalmoleküle bei Gram-negativen und Gram-positiven Keimen identifiziert [Miller, M.B., Bassler, B.L. (2001) Quorum sensing in bacteria. Annu Rev Microbiol. 55, 165-199; Kleerebezem, M., Quadri, L.E. (2001) Peptide pheromone-dependent regulation of antimicrobial peptide production in Gram-positive bacteria: a case of multicellular behavior. Peptides 22, 1579-1596].

Die Struktur dieser Signalmoleküle ist insbesondere bei Gram-negativen Organismen sehr gut untersucht. Bei den von diesen Organismen eingesetzten Signalstoffen (Pheromonen) handelt es sich häufig um Vertreter aus einer Gruppe verschiedener N-Acyl-L-Homoserinlactone (AHL), die sich durch die Länge der N-Acyl-Seitengruppe sowie durch Modifikationen der C-3-Position (3-Oxo- oder 3-Hydroxygruppe) unterscheiden [Greenberg, E.P. (1997) Quorum sensing in Gram-negative bacteria. ASM News 63, 371-377].

Bei Gram-positiven Bakterien sind die Mechanismen noch nicht so gut bekannt. Häufig sind hier kleinere Peptide als Signalmoleküle beschrieben. [Kleerebezem, M., Quadri, L.E. (2001) Peptide pheromone-dependent regulation of antimicrobial peptide production in Gram-positive bacteria: a case of multicellular behavior. Peptides 22, 1579-1596].

Das Grundprinzip ist jedoch ähnlich. Das jeweilige Signalmolekül wird von spezifischen, in der Regel membrangebundenen, zellulären Rezeptoren erkannt und gebunden (Zwei-Komponentensysteme).

Bei Gram-negativen Bakterien fungieren u.a. spezielle Histidin-Kinasen als besagte Rezeptoren. Diese Histidin-Kinasen sind Bestandteile sogenannter Zwei-Komponenetensysteme. Die zweite Komponente wird jeweils durch den sogenannten Response-Regulator gebildet. Wenn ein Signalmolekül an eine solche Histidin-Kinase bindet, wird von dieser der Response-Regulator aktiviert. Der aktivierte Response-Regulator fungiert dann seinerseits als Aktivator verschiedenster zellulärer Prozesse [Chang C, Stewart, The two-component system. Regulation of diverse signaling pathways in prokaryotes and eukaryotes. Plant Physiol. 1998 Jul;117(3):723-31.]

Während es in allen Zellen eine Vielzahl solcher Zwei-Komponenten-Systeme mit den unterschiedlichsten Funktionen gibt, sind für die Biofilmbildung durch Gram-negative Bakterien hauptsächlich intrazelluläre Transkriptions-Aktivator-Proteine ("LuxR-Proteine") verantwortlich. Von ihrer Funktionalität sind die Proteine der LuxR- Klasseh zum einen in der Lage, AHLs zu binden, andererseits aber auch Wechselwirkungen mit Steuerungsbereichen von DNA-Molekülen einzugehen. [Eberl L, N-acyl homoserinelactone-mediated gene regulation in gram-negative bacteria, Syst Appl Microbiol. 1999 Dec;22(4):493-506.; Michael B, Smith JN, Swift S, Heffron F, Ahmer BM. SdiA of Salmonella enterica is a LuxR homolog that detects mixed microbial communities. J Bacteriol. 2001 Oct;183(19):5733-42.; Gray KM, Garey JR., The evolution of bacterial Luxl and LuxR quorum sensing Microbiology. 2001 Aug;147(Pt 8):2379-87.].

Seit kurzem ist bekannt, daß für die Bindung der AHL-Moleküle nur der N-terminale Teil des Transkriptions-Aktivator-Proteins (ca. 200 Aminosäuren) verantwortlich ist. [Zhang RG, Pappas T, Brace JL, Miller PC, Oulmassov T, Molyneaux JM, Anderson JC, Bashkin JK, Winans SC, A., Structure of a bacterial quorum-sensing transcription factor complexed with pheromone and DNA. Nature. 2002 Jun 27;417(6892):971-4.].

Aus dem Stand der Wissenschaft und Technik sind im Wesentlichen drei unterschiedliche Konzepte zur Biofilmverhinderung bzw. -kontrolle beschrieben:

### 1. Spaltung der Botenstoffe durch Enzyme z.B. Lactonasen.

Kürzlich wurden verschiedene Enzyme aus gram-positiven Bacillen isoliert, die AHL-Moleküle spezifisch spalten und somit diese Signalwege ausschalten können ("Quorum Quenching"). Diese Lactonasen sind mehrfach beschrieben worden [Dong YH, Wang LH, Xu JL, Zhang HB, Zhang XF, Zhang LH. (2001) Quenching quorum-sensing-dependent bacterial infection by an N-acyl homoserine lactonase. Nature. 411, 813-817; WO 0185664; WO 0216623].

### 2. Blockierung/Bindung der Botenstoffe selbst z.B. mit Antikörpern.

Eine weitere Möglichkeit zur Verhinderung von Biofilmen ist der Einsatz von AHLspezifischen Antikörpern. Nach Bindung eines solchen Antikörpers an den Botenstoff, ist dieser nicht mehr in der Lage an seinen ursprünglichen Rezeptor (=Histidin-Kinase) zu binden [W00194543]. Auf Grund der hohen Kosten für die Antikörperflerstellung ist diese Methode allerdings bestenfalls für medizinische Zwecke praktikabel.

### 3. Blockierung der zellulären Rezeptoren der Botenstoffe z.B. durch strukturanaloge Substanzen.

Als strukturanaloge Substanzen sind hier insbesondere Furanone und Furanonderivate bekannt. [Manefield M, de Nys R, Kumar N, Read R, Givskov M, Steinberg P, Kjelleberg., Evidence that halogenated furanones from Delisea pulchra inhibit acylated homoserine lactone (AHL)-mediated gene expression displacing the AHL signal from its receptor protein. Microbiology. 1999 Feb;145 ( Pt 2):283-91; WO9629392; WO0168091; WO0168090; W00176594].

Furanone stellen hinsichtlich ihrer Struktur AHL-Analoga dar. Sie unterbrechen den Signalübertragungsweg, indem sie die AHL-Signalstoffe kompetitiv verdrängen und selbst an das intrazelluläre Zielmolekül (intrazelluläreTranskriptions-Aktivator-Protein) binden. Das Wachstum der betroffenen Keime wird durch die Furanon-Einwirkung bei niedrigen Konzentrationen nicht gehemmt.

Neben dem Einsatz von Furanonen wird in Einzelfällen auch die Verwendung von Antikörpern bzw. Antikörperfragmenten erwähnt. [Bryers, J.D., 2001, Gene therapy approach to preventing bacterial colonization of biomaterials, Abstracts of papers, 222nd ACS National Meeting]

Der vorliegenden Erfindung liegt die Aufgabe zugrunde, neue Substanzen bereitzustellen, mittels derer die Interaktion von Mikroben untereinander gesteuert und reguliert werden kann.

Gegenstand der vorliegenden Erfindung ist daher eine Verbindung der Formel I, 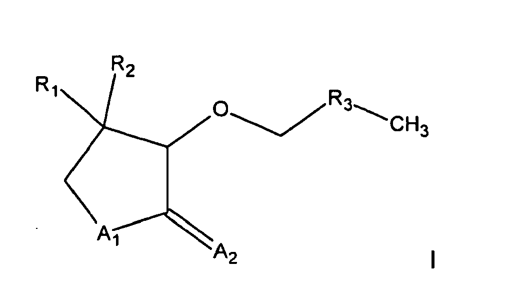
in der A₁ für O oder NH steht,
A₂ für O oder S steht,
R₁ und R₂ unabhängig voneinander für einen Rest stehen, der ausgewählt ist unter Wasserstoff, Methyl-, oder C₂-C₈-, insbesondere C₂-C₆-, vor allem C₂-C₄-, gesättigten oder ein- oder zweifach ungesättigten, verzweigten oder linearen Kohlenwasserstoffresten,
R₃ für einen Rest steht, der ausgewählt ist unter C₃-C₁₈-, C₃-C₁₆- oder C₃-C₁₄- gesättigten oder ein- oder zweifach ungesättigten, verzweigten oder linearen Kohlenwasserstoffresten.

Vorzugsweise stehen A₁ und A₂ für O, R₁ und R₂ für Wasserstoff oder Methyl, und R₃ steht vorzugsweise für Butylen, Pentylen, Hexylen, Heptylen, Octylen, Nonylen, Decylen, Undecylen, Dodecylen, Tridecylen oder Tetradecylen. In einer besonders bevorzugten Ausführungsform stehen R₁ und R₂ für Wasserstoff und R₃ für Decylen, Undecylen, Dodecylen, Tridecylen oder Tetradecylen. In einer weiteren besonders bevorzugten Ausführungsform stehen R₁ und R₂ für Methyl und R₃ für Butylen, Pentylen, Hexylen, Heptylen oder Octylen.

Erfindungsgemäß ganz besonders bevorzugt sind α-Octyloxy-Pantolacton, α-Octyloxy-Butyrolacton, α-Tridecyloxy-Butyrolacton, α-Hexadecyloxy-Butyrolacton, α-Heptyloxy-Pantolacton, α-Tridecyloxy-Pantolacton und α-HexadecyloxyPantolacton.

Die Kohlenwasserstoffreste der Verbindung gemäß Formel I, und insbesondere unabhängig voneinander die Reste R₁, R₂ und R₃ können gegebenenfalls auch ein Heteroatom ausgewählt aus O und S in der Kette enthalten und/oder ein- oder mehrfach, vorzugsweise einfach, substituiert sein, insbesondere durch Reste ausgewählt aus Halogen, insbesondere Fluor, Chlor oder Brom, Hydroxy, C₁-C₆-Alkyl, insbesondere C₁-C₄-Alkyl, Trifluormethyl, C₁-C₆-Alkoxy, insbesondere C₁-C₄-Alkoxy, vorzugsweise Methoxy oder Ethoxy, C₆-C₁₀-Aryl, insbesondere C₆-Aryl, vorzugsweise Phenyl, und C₁-C₆-Alkyl-C₆-C₁₀-aryl, insbesondere C₁-C₄-Alkyl-C₆-Aryl, vorzugsweise Toloyl.

Die erfindungsgemäßen Verbindungen können in Form ihres Racemats oder in Form ihrer isolierten Stereoisomere und/oder Enantiomere vorliegen.

Die erfindungsgemäßen Verbindungen lassen sich vorzugsweise durch Umsetzung des entsprechenden Lactons, Lactams oder Thiolactams mit einem 1-Halogenalkan, insbesondere 1-Chloralkan, 1-Bromalkan oder 1-lodalkan, besonders bevorzugt 1-Bromalkan, in einem geeigneten organischen Lösungsmittel (z. B. Dimethylformamid) in Anwesenheit einer geeigneten Base, insbesondere Cäsiumcarbonat oder Cäsiumhalogenid, herstellen.

Erfindungsgemäße Verbindungen, in denen R₃ ein Heptylenrest oder ein kürzerer Rest ist, lassen sich durch Destillation aus dem Reaktionsgemisch aufarbeiten. Ist R₃ ein Octylen- oder ein größerer Rest, erfolgt die Aufarbeitung vorzugsweise durch Umkristallisation.

Die Herstellungsverfahren können durch den Fachmann in geeigneter Weise modifiziert werden, abhängig von der Größe und Art der gewünschten Zielverbindung.

Ein weiterer Gegenstand der vorliegenden Erfindung ist ein Verfahren zur Kontrolle von auf mikrobieller Interaktion beruhenden Vorgängen mit Ausnahme von Verfahren zur therapeutischen Behandlung des menschlichen oder tierischen Körpers, dadurch gekennzeichnet, daß man
a) gegebenenfalls die interagierenden Mikroorganismen bestimmt,
b) gegebenenfalls unter den erfindungsgemäßen Verbindungen die geeignete Verbindung oder die geeigneten Verbindungen auswählt, und
c) die erfindungsgemäßen Verbindungen in für die gewünschte Kontrolle ausreichender Menge dem Medium zusetzt, in dem die mikrobielle Interaktion stattfindet.

Besonders geeignete Mikroorganismen sind ausgewählt unter *Aeromonas hydrophila, Aeromonas salmonicida, Agrobacterium tumefaciens, Burkholderia cepacia, Chromobacterium violaceum, Enterobacter agglomerans, Erwinia carotovora, Erwinia chrysanthemi, Escherichia coli, Nitrosomona europaea, Obesumbacterium proteus, Pantoea stewartii, Pseudomonas aeruginosa, Pseudomonas aureofaciens, Pseudomonas fluorescens, Pseudomonas syringae, Ralstonia solanacearum, Rhizobium etli, Rhizobium leguminosarum, Rhodobacter sphaeroides, Salmonella enterica, Serratia liquefaciens, Vibrio anguillarum, Vibrio fischeri, Xenorhabdus nematophilus, Yersinia enterolytica, Yersinia pestis, Yersinia pseudotuberculosis* und *Yersinia ruckeri.*

Erfindungsgemäß sind auch Gram-positive Bakterien geeignete Mikroorganismen.

Am bedeutsamsten sind Keime, die insbesondere in wässrigen Lösungen stark an der Biofilmbildung beteiligt sind. Das sind in erster Linie Keime aus der großen Pseudomonaden-Gruppe, insbesondere P. aeruginosa, Burkholderia cepacia, Serratia, Rhizobium, E. coli. Weitere wichtige Biofilm-Keime sind Aquabakterium und Xanthomonas.

In den meisten Fällen steuern die bakteriellen Kommunikationssysteme nicht morphologische Veränderungen einzelner Zellen, sondern beeinflussen die Pathogenität der jeweiligen Organismen. Einige der bedeutsamsten Funktionen dieser Kommunikationssysteme sind nachfolgend beispielhaft aufgelistet:
- Steuerung der Expression der Biolumineszenzgene (z. B. Photobacterium fischeri)
- Produktion des ß-Lactam-Antibiotikums Carbapenem (Erwinia carotovora) und AB-Produktion in Pseudomonas aureofaciens
- Konjugativer Plasmidtransfer (tral/traR aus Agrobacterium tumefaciens),
- Starvation response (z. B. Pseudomonas)
- Bakterielle Fortbewegung (swrl/swrR aus Serratia liquefaciens) und bei P. aeruginosa
- Ausbildung differenzierter Biofilme (P aeruginosa, B. cepacia)
- Produktion verschiedenster Virulenzfaktoren (zellassoziierte Virulenzfaktoren bei P. aeruginosa, z.B. extrazelluläre Faktoren, wie Proteasen (LasB-Elastase, alkalische Protease und LasA-Protease), Hämolysine (Rhamnolipid und Phosoholipase) und Toxine (Exotoxin A und Exoenzym S)
- Interspezifische Zell-Zell-Kommuniation ("cross-talk" zwischen verschiedenen Bakterienspezies) z. B. zwischen P. cepacia und P. aeruginosa

Die Regulation der Produktion von Virulenzfaktoren spielt insbesondere bei Pseudomonas aeruginosa und Burkholderia cepacia im Zusammenhang mit der chronischen Infektion von Mukoviszidosepatienten eine wichtige Rolle

Erfindungsgemäß ist die mikrobielle Interaktion ausgewählt unter der Ausbildung und/oder Reifung von Biofilmen, multizellulärem Schwärmverhalten, der konzertierten Ausbildung von Antibiotika-Resistenzen, der konzertierten Synthese von Antibiotika, der konzertierten Synthese von Pigmentstoffen, der konzertierten Produktion extrazellulärer Enzyme, insbesondere hydrolytischer Enzyme, sowie der konzertierten Produktion von Virulenzfaktoren, vorzugsweise der Ausbildung und/oder Reifung von Biofilmen.

Ein weiterer Gegenstand der Erfindung ist die Verwendung der erfindungsgemäßen Verbindungen zur Kontrolle von auf mikrobieller Interaktion beruhenden Vorgängen, insbesondere zur Kontrolle der Ausbildung und/oder Reifung von Biofilmen, besonders bevorzugt von Biofilmen, an denen Gram-negative Bakterien beteiligt sind, mit Ausnahme von Verwendungen zur therapeutischen Behandlung des menschlichen oder tierischen Körpers.

Beispielsweise lassen sich so Schiffsrümpfe vor Algenbewuchs schützen. Denn der Biofilm bildet die Grundlage für die Ansiedlung von größeren Organismen wie Muscheln und Algen. Dieser Bewuchs bremst durch seinen Reibungswiderstand die Schiffe und treibt somit den Treibstoffverbrauch in die Höhe, weshalb der Belag regelmäßig aufwendig entfernt werden muss.

Vorteilhafterweise töten die erfindungsgemäßen Verbindungen im Gegensatz zu Antibiotika die Bakterien nicht ab, sondern inhibieren nur ihr Kommunikationssystem, so dass es zu keiner Ausbildung eines reifen, schleimigen Biofilms kommt. Besonders vorteilhaft ist, daß die Mikroorganismen keine Resistenzen gegen diese Verbindungen ausbilden können, die Strukturanaloga zu den von ihnen selbst verwendeten AHL-Molekülen darstellen, da sie ansonsten Ihr eigenes Kommunikationsystem stark beeinträchtigen würden.

Medizinisch relevante Biofilmen sind ein besonders bevorzugtes Ziel der vorliegenden Erfindung. Zu nennen sind hier insbesondere:

Mukoviszidose: Die chronische Lungeninfektion, die Mukoviszidose-Patienten befällt, wird von dem gram-negativen Stäbchenbakterium Pseudomonas aeruginosa verursacht. Sobald sich ein Biofilm auf der Lunge gebildet hat, sind die Bakterien selbst durch aggressivste Antibiotika-Behandlungen nicht mehr zu vernichten. Patienten mit dieser Erbkrankheit bilden auf Grund eines gestörten Salztransports in den Epithelzellen, zähflüssigen Schleim in den Lungen aus, der einen guten Nährboden für Erreger bildet.

Kontaktlinsen: Auch auf Kontaktlinsen können sich filmbildende Bakterien niederlassen. Vor allem der Keim Pseudomonas aeruginosa spielt hier eine wichtige Rolle. Obwohl er in der normalen Flora des Auges nicht vorkommt, kann er durch Mascara-Schwämmchen oder kontaminierte Reinigungslösungen für Kontaktlinsen ins Auge gelangen. Zu Hornhautentzündungen kommt es häufig, wenn auch nur kleine Verletzungen vorliegen.

Implantate: Bakterielle Biofilme sind für etwa 60 Prozent aller Infektionen in der Implantationschirurgie verantwortlich. Die Mortalität der Patienten ist besonders hoch, wenn endogene Implantate wie künstliche Gelenke oder Herzklappen betroffen sind.

Katheter: Intravenöse Zugänge, wie sie für Bluttransfusionen oder künstliche Ernährung benötigt werden, können auch zu schweren Infektionen führen. Keime der normalen Hautflora wie Staphylococcus-Arten oder Erreger wie verschiedene Pseudomonas-Spezies können sich an der Außenseite des Zugangs anlagern, bevor er in die Blutgefäße des Patienten eingeführt wird. Dort bilden die Bakterien dann einen Film, der, wenn er sich ablöst, chronische Infektionen auslösen kann.

Zahnbelag: Der Belag auf Zähnen ist nicht nur unschön, sondern unter Umständen auch gefährlich: Karies, Gingivitis (Zahnfleischbluten) und Paradontitis (Zahnfleischentzündung) können die Folge sein. Außerdem können Keime der Mundflora durch kleine Wunden in das Blutsystem gelangen, die im Verdacht stehen, Herzinfarkte, Frühgeburten oder Diabetes zu verursachen.

Bevorzugtermaßen erfolgt die erfindungsgemäße Verwendung daher in Sterilisations-, Desinfektions- Imprägnier- oder Konservierungsmitteln, Wasch- oder Reinigungsmitteln, oder in Kühl- oder Kühlschmiermitteln (technische Anwendungslösungen) sowie auf dem Gebiet der Wasserreinigung / Wasserbehandlung sowie der Arzneimittel,- Lebensmittel-, Brauerei-, Medizintechnik-,Farben-, Holz-, Textil-, Kosmetik-, Leder-, Tabak-, Pelz-, Seil-, Papier-, Zellstoff-, Kunststoff-, Treibstoff-, Öl-, Kautschuk- oder Maschinenindustrie.

Besonders bevorzugt ist die erfindungsgemäße Verwendung zur Biofilmkontrolle somit bei medizinischen Geräten, Instrumenten und Apparaturen, insbesondere bei Kathetern und Endoskopen.

Weitere Gegenstände der vorliegenden Erfindung sind Körperpflegemittel, Haarwaschmittel, Haarpflegemittel, Schaumbäder, Duschbäder, Cremes, Gele, Lotionen, alkoholische und wässrig/alkoholische Lösungen, Emulsionen, Wachs/ Fett-Massen, Stiftpräparate, Puder oder Salben; Mund-, Zahn- oder Zahnprothesenpflegemittel, Kosmetika, Waschmittel, Reinigungsmittel, Nachspülmittel, Handwaschmittel, Handgeschirrspülmittel, Maschinengeschirrspülmittel, Desinfektionsmittel und Mittel zur Behandlung von Lebensmitteln, Arzneimitteln, Filtermedien, Textilien, Pelzen, Papier, Fellen oder Leder, enthaltend erfindungsgemäße Verbindungen.

Die Haarwasch- und/oder Haarpflegemittel sowie Schaumbäder, Duschbäder, Cremes, Gele, Lotionen, alkoholische und wässrig/alkoholische Lösungen, Emulsionen, Wachs/ Fett-Massen, Stiftpräparate, Puder oder Salben, die erfindungsgemäßen Verbindungen umfassen, können als Hilfs- und Zusatzstoffe milde Tenside, Ölkörper, Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deodorantien, Antitranspirantien, Antischuppenmittel, Filmbildner, Quellmittel, UV-Licht-schutzfaktoren, Antioxidantien, Hydrotrope, Konservierungsmittel, Insektenrepellentien, Selbstbräuner, Solubilisatoren, Parfümöle, Farbstoffe und dergleichen enthalten.

Typische Beispiele für geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, □-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

Als Emulgatoren kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren E-thylenoxidanlagerungsprodukte;
(4) Alkyl- und/oder Alkenylmono- und -oligoglycoside mit 8 bis 22 Kohlenstoffatomen im Alk(en)ylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEGalkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß DE 1165574 PS und/oder Mischester von Fettsäuren mit 6 bis 22 Kohlenstoffatomen, Methylglucose und Polyolen, vorzugsweise Glycerin oder Polyglycerin,
(13) Polyalkylenglycole sowie
(14) Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE 2024051 PS als Rückfettungsmittel für kosmetische Zubereitungen bekannt.

Alkyl- und/oder Alkenylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Perlglanzwachse kommen beispielsweise in Frage: Alkylenglycolester, speziell Ethylenglycoldisfiearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von. Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete kationische Polymere sind beispielsweise kationische Cell ulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte VinylpyrrolidonNinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der FR 2252840 A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Januar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproyl-methacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm.Toil. 91, 27 (1976).

Typische Beispiele für Fette sind Glyceride, als Wachse kommen u.a. natürliche Wachse, wie z.B. Candelillawachs, Carnaubawachs, Japanwachs, Espartograswachs, Korkwachs, Guarumawachs, Reis-keimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als Stabilisatoren können Metallsalze von Fettsäuren, wie z.B. Magnesium-, Aluminium- und/oder Zinkstearat bzw. -ricinoleat eingesetzt werden.

Unter biogenen Wirkstoffen sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als keimhemmende Mittel, die gegebenenfalls den erfindungsgemäßen Kosmetika zugesetzt werden können, sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Menthol, Minzöl, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Enzyminhibitoren können den erfindungsgemäßen Kosmetika ebenfalls zugesetzt werden. Beispielsweise sind Esteraseinhibitoren möglicherweise geeignete Enzyminhibitoren. Hierbei handelt es sich vorzugsweise um Trialkylcitrate wie Trimethylcitrat, Tripropylcitrat, Trüsopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder - phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe.

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
(a) adstringierende Wirkstoffe,
(b) Ölkomponenten,
(c) nichtionische Emulgatoren,
(d) Coemulgatoren,
(e) Konsistenzgeber,
(f) Hilfsstoffe wie z. B. Verdicker oder Komplexierungsmittel und/oder
(g) nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin.

Daneben können in Antitranspirantien übliche öllösliche und wasserlösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als Antischuppenmittel können Climbazol, Octopirox und Zinkpyrethion eingesetzt werden.

Gebräuchliche Filmbildner sind beispielsweise Chitosan, mikrokristallines Chitosan, quaterniertes Chitosan, Polyvinylpyrrolidon, Vinylpyrrolidon-VinylacetatCopolymerisate, Polymere der Acrylsäurereihe, quaternäre Cellulose-Derivate, Kollagen, Hyaluronsäure bzw. deren Salze und ähnliche Verbindungen.

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993) entnommen werden.

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher wie in der EP 0693471 B1 beschrieben;
- 4-Aminobenzoesäurederivate, vorzugsweise 4-Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
- Propan-1,3-dione, wie z.B. 1-(4-tert.Butylphenyl)-3-4'methoxyphenyl)propan-1,3-dion;
- Ketotricyclo(5.2.1.0)decan-Derivate, wie in der EP 0694521 B1 beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)-sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996) zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als Insekten-Repellentien kommen N,N-Diethyl-m-toluamid, 1,2-Pentandiol oder Ethyl Butylacetylaminopropionate in Frage, als Selbstbräuner eignet sich Dihydroxyaceton.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe.

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind.

Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

Die erfindungsgemäßen Mund-, Zahn- und/oder Zahnprothesenpflegemittel können beispielsweise als Mundwasser, Gel, flüssige Zahnputzlotion, steife Zahnpaste, Gebissreiniger oder Prothesenhaftcreme vorliegen.

Hierzu ist es erforderlich, die erfindungsgemäßen Verbindungen in einen geeigneten Träger einzuarbeiten.

Als Träger können z.B. auch pulverförmige Zubereitungen oder wässrigalkoholische Lösungen dienen, die als Mundwässer 0 bis 15 Gew.-% Ethanol, 1 bis 1,5 Gew.-% Aromaöle und 0,01 bis 0,5 Gew.-% Süßstoffe oder als Mundwasser-Konzentrate 15 bis 60 Gew.-% Ethanol, 0,05 bis 5 Gew.-% Aromaöle, 0,1 bis 3 Gew.-% Süßstoffe sowie ggf. weitere Hilfsstoffe enthalten können und vor Gebrauch mit Wasser verdünnt werden. Die Konzentration der Komponenten muss dabei so hoch gewählt werden, dass nach Verdünnung die genannten Konzentrationsuntergrenzen bei der Anwendung nicht unterschritten werden.

Als Träger können aber auch Gele sowie mehr oder weniger fließfähige Pasten dienen, die aus flexiblen Kunststoffbehältern oder Tuben ausgedrückt und mit Hilfe einer Zahnbürste auf die Zähne aufgetragen werden. Solche Produkte enthalten höhere Mengen an Feuchthaltemitteln und Bindemitteln oder Konsistenzreglern und Polierkomponenten. Darüber hinaus sind auch in diesen Zubereitungen Aromaöle, Süßstoffe und Wasser enthalten.

Als Feuchthaltemittel können dabei z.B. Glycerin, Sorbit, Xylit, Propylenglykole, Polyethenylenglycole oder Gemische dieser Polyole, insbesondere solche Polyethenylenglycole mit Molekulargewichten von 200 bis 800 (von 400 - 2000) verwendet werden enthalten sein. Bevorzugt ist als Feuchthaltemittel Sorbit in einer Menge von 25 - 40 Gew.-% enthalten.

Als Antizahnstein-Wirkstoffe und als Demineralisierungs-Inhibitoren können kondensierten Phosphate in Form ihrer Alkalisalze, bevorzugt in Form ihrer Natrium- oder Kaliumsalze enthalten sein. Die wäßrigen Lösungen dieser Phosphate reagieren aufgrund hydrolytischer Effekte alkalisch. Durch Säurezusatz wird der pH-Wert der erfindungsgemäßen Mund-, Zahn- und/oder Zahnprothesenpflegemittel auf die bevorzugten Werte von 7,5 - 9 eingestellt.

Es können auch Gemische verschiedener kondensierter Phosphate oder auch hydratisierte Salze der kondensierten Phosphate eingesetzt werden. Die spezifizierten Mengen von 2 - 12 Gew.-% beziehen sich jedoch auf die wasserfreien Salze. Bevorzugt ist als kondensiertes Phosphat ein Natrium- oder Kaliumtripolyphosphat in einer Menge von 5 - 10 Gew.-% der Zusammensetzung enthalten.

Ein bevorzugt enthaltener Wirkstoff ist eine karieshemmende Fluorverbindung, bevorzugt aus der Gruppe der Fluoride oder Monofluorophosphate in einer Menge von 0,1 - 0,5 Gew.-% Fluor. Geeignete Fluorverbindungen sind z.B. Natriummonofluorophosphat (Na₂PO₃F) Kaliummonofluorophosphat, Natrium- oder Kaliumfluorid, Zinnfluorid oder das Fluorid einer organischen Aminoverbindung.

Als Bindemittel und Konsistenzregler dienen z.B. natürliche und synthetische wasserlösliche Polymere wie Carragheen, Traganth, Guar, Stärke und deren nichtionogene Derivate wie z.B. Hydroxypropylguar, Hydroxyethylstärke, Celluloseether wie z.B. Hydroxyethylcellulose oder Methylhydroxypropylcellulose. Auch Agar-Agar, Xanthan-Gum, Pektine, wasserlösliche Carboxyvinylpolymere (z.B. Carbopol^{®}-Typen), Polyvinylalkohol, Polyvinylpyrrolidon, höhermolekulare Polyethylenglykole (Molekulargewicht 10³ bis 10⁶ D). Weitere Stoffe, die sich zur Viskositätskontrolle eignen, sind Schichtsilikare wie z.B. Montmorillonit-Tone, kolloidale Verdickungskieselsäuren, z.B. Aerogel-Kieselsäure oder pyrogene Kieselsäuren.

Als Polierkomponenten können alle hierfür bekannten Poliermittel, bevorzugt aber Fällungs- und Gelkieselsäuren, Aluminiumhydroxid, Aluminiumsilicat, Aluminiumoxid, Aluminiumoxidtrihydrat, unlösliches Natriummetaphosphat, Calciumpyrophosphat, Calciumhydrogenphosphat, Dicalciumphosphat, Kreide, Hydroxylapatit, Hydrotalcite, Talkum, Magnesiumaluminiumsilicat (Veegum^{®}), Calciumsulfat, Magnesiumcarbonat, Magnesiumoxid, Natriumaluminiumsilikate, z.B. Zeolith A oder organische Polymere, z.B. Polymethacrylat, eingesetzt werden. Die Poliermittel werden vorzugsweise in kleineren Mengen von z.B. 1 - 10 Gew.-% verwendet.

Die erfindungsgemäßen Zahn- und/oder Mundpflegeprodukte können durch Zugabe von Aromaölen und Süßungsmitteln in ihren organoleptischen Eigenschaften verbessert werden. Als Aromaöle kommen alle für Mund-, Zahn- und/oder Zahnprothesenpflegemittel gebräuchlichen natürlichen und synthetischen Aromen in Frage. Natürliche Aromen können sowohl in Form der aus den Drogen isolierten etherischen Öle als auch der aus diesen isolierten Einzelkomponenten verwendet werden. Bevorzugt sollte wenigstens ein Aromaöl aus der Gruppe Pfefferminzöl, Krauseminzöl, Anisöl, Kümmelöl, Eukalyptusöl, Fenchelöl, Zimtöl, Geraniumöl, Salbeiöl, Thymianöl, Majoranöl, Basilikumöl, Citrusöl, Gaultheriaöl oder eine oder mehrere daraus isolierte synthetisch erzeugten Komponenten dieser Öle enthalten sein. Die wichtigsten Komponenten der genannten Öle sind z.B. Menthol, Carvon, Anethol, Cineol, Eugenol, Zimtaldehyd, Geraniol, Citronellol, Linalool, Salven, Thymol, Terpinen, Terpinol, Methylchavicol und Methylsalicylat. Weitere geeignete Aromen sind z.B. Menthylacetat, Vanillin, Jonone, Linalylacetat, Rhodinol und Piperiton. Als Süßungsmittel eignen sich entweder natürliche Zucker wie Sucrose, Maltose, Lactose und Fructose oder synthetische Süßstoffe wie z.B. Saccharin-Natriumsalz, Natriumcyclamat oder Aspartam.

Als Tenside sind dabei insbesondere Alkyl- und/oder Alkenyl-(oligo)-glycoside einsetzbar. Ihre Herstellung und Verwendung als oberflächenaktive Stoffe sind beispielsweise aus US-A-3 839 318, US-A-3 707 535, US-A-3 547 828 DE-A-19 43 689, DE-A-20 36 472 und DE-A-30 01 064 sowie EP-A-77 167 bekannt. Bezüglich des Glycosidrestes gilt, daß sowohl Monoglycoside (x = 1), bei denen ein Pentose- oder Hexoserest glycosidisch an einen primären Alkohol mit 4 bis 16 C-Atomen gebunden ist, als auch oligomere Glycoside mit einem Oligomerisationsgrad x bis 10 geeignet sind. Der Oligomerisationsgrad ist dabei ein statistischer Mittelwert, dem eine für solche technischen Produkte übliche Homologenverteilung zugrunde liegt.

Bevorzugt eignet sich als Alkyl- und/oder Alkenyl-(oligo)-glycosid ein Alkyl- und/oder Alkenyl-(oligo)-glucosid der Formel RO(C₆H₁₀O)ₓ-H, in der R eine Alkyl- und/oder Alkenyl-gruppe mit 8 bis 14 C-Atomen ist und x einen Mittelwert von 1 bis 4 hat. Besonders bevorzugt sind Alkyloligoglucoside auf Basis von gehärtetem C_{12/14}-Kokosalkohol mit einem DP von 1 bis 3. Das Alkyl- und/oder Alkenylglycosid-Tensid kann sehr sparsam verwendet werden, wobei bereits Mengen von 0,005 bis 1 Gew.-% ausreichend sind.

Außer den genannten Alkylglucosid-Tensiden können auch andere nichtionische, ampholytische und kationische Tenside enthalten sein, als da beispielsweise sind: Fettalkoholpolyglycolethersulfate, Monoglyceridsulfate, Monoglyceridethersulfate, Mono- und/oder Dialkylsulfosuccinate, Fettsäureisethionate, Fettsäuresarcosinate, Fettsäuretauride, Fettsäureglutamate, Ethercarbonsäuren, Fettsäureglucamide, Alkylamido-betaine und/oder Proteinfettsäurekondensate, letztere vorzugsweise auf Basis von Weizenproteinen. Insbesondere zur Solubilisierung der meist wasserunlöslichen Aromaöle kann ein nichtionogener Lösungsvermittler aus der Gruppe der oberflächenaktiven Verbindungen erforderlich sein. Besonders geeignet für diesen Zweck sind z.B. oxethylierte Fettsäureglyceride, oxethylierte Fettsäuresorbitanpartialester oder Fettsäurepartialester von Glycerin- oder Sorbitan-Oxethylaten. Lösungsvermittler aus der Gruppe der oxethylierten Fettsäureglyceride umfassen vor allem Anlagerungsprodukte von 20 bis 60 Mol Ethylenoxid an Mono- und Diglyceride von linearen Fettsäuren mit 12 bis 18 C-Atomen oder an Triglyceride von Hydroxyfettsäuren wie Oxystearinsäure oder Ricinolsäure. Weitere geeignete Lösungsvermittler sind oxethylierte Fettsäuresorbitanpartialester; das sind bevorzugt Anlagerungsprodukte von 20 bis 60 Mol Ethylenoxid an Sorbitanmonoester und Sorbitandiester von Fettsäuren mit 12 bis 18 C-Atomen. Ebenfalls geeignete Lösungsvermittler sind Fettsäurepartialester von Glycerin- oder Sorbitan-Oxethylaten; das sind bevorzugt Mono- und Diester von C₁₂-C₁₈-Fettsäuren und Anlagerungsprodukten von 20 bis 60 Mol Ethylenoxid an 1 Mol Glycerin oder an 1 Mol Sorbit.

Die erfindungsgemäßen Mund-, Zahn- und/oder Zahnprothesenpflegemittel enthalten bevorzugt als Lösungsvermittler für gegebenenfalls enthaltene Aromaöle Anlagerungsprodukte von 20 bis 60 Mol Ethylenoxid an gehärtetes oder ungehärtetes Rizinusöl (d.h. an Oxystearinsäure- oder Ricinolsäure-triglycerid), an Glyzerin-mono- und/oder -distearat oder an Sorbitanmono- und/oder -distearat.

Weitere übliche Zusätze für die Mund-, Zahn- und/oder Zahnprothesenpflege mittel sind z.B.
- Pigmente, z.B. Titandioxid, und/oder Farbstoffe
- pH-Stellmittel und Puffersubstanzen wie z.B. Natriumbicarbonat, Natriumcitrat, Natriumbenzoat, Zitronensäure, Phosphorsäure oder saure Salze, z.B. NaH₂PO₄
- wundheilende und entzündungshemmende Stoffe wie z.B. Allantoin, Harnstoff, Panthenol, Azulen bzw. Kamillenextrakt
- weitere gegen Zahnstein wirksame Stoffe wie z.B. Organophosphonate, z.B. Hydroxyethandiphosphonate oder Azacycloheptandiphosphonat
- Konservierungsstoffe wie z.B. Sorbinsäure-Salze, p-HydroxybenzoesäureEster.
- Plaque-Inhibitoren wie z.B. Hexachlorophen, Chlorhexidin, Hexetidin, Triclosan, Bromchlorophen, Phenylsalicylsäureester.

In einer besonderen Ausführungsform ist die Zusammensetzung eine Mundspülung, ein Mundwasser, ein Prothesenreiniger oder ein Prothesenhaftmittel.

Für erfindungsgemäß bevorzugten Prothesenreiniger, insbesondere Prothesenreinigungstabletten und -pulver, eignen sich neben den schon genannten Inhaltsstoffen für die Mund-, Zahn- und/oder Zahnprothesenpflege zusätzlich noch PerVerbindungen wie beispielsweise Peroxoborat, Peroxomonosulfat oder Percarbonat. Sie haben den Vorteil, dass sie neben der Bleichwirkung gleichzeitig auch desodorierend und/oder desinfizierend wirken. Der Einsatz solcher PerVerbindungen in Prothesenreinigern beträgt zwischen 0,01 und 10 Gew.-%, insbesondere zwischen 0,5 und 5 Gew.-%.

Als weitere Inhaltsstoffe sind auch Enzyme, wie z.B. Proteasen und Carbohydrase, zum Abbau von Proteinen und Kohlenhydraten geeignet. Der pH-Wert kann zwischen pH 4 und pH 12, insbesondere zwischen pH 5 und pH 11 liegen.

Für die Prothesenreinigungstabletten sind zusätzlich noch weitere Hilfsstoffe notwendig, wie beispielsweise Mittel, die einen sprudelnden Effekt hervorrufen, wie z.B. CO₂ freisetzende Stoffe wie Natriumhydrogencarbonat, Füllstoffe, z.B. Natriumsulfat oder Dextrose, Gleitmittel, z.B. Magnesiumstearat, Fließregulierungsmittel, wie beispielsweise kolloidales Siliziumdioxid und Granuliermittel, wie die bereits erwähnten hochmolekularen Polyethylenglykole oder Polyvinylpyrrolidon.

Prothesenhaftmittel können als Pulver, Cremes, Folien oder Flüssigkeiten angeboten werden und unterstützen die Haftung der Prothesen.

Als Wirkstoffe sind natürliche und synthetische Quellstoffe geeignet. Als natürliche Quellstoffe sind neben Alginaten auch Pflanzengummen, wie z.B. Gummi arabicum, Traganth und Karaya-Gummi sowie natürlicher Kautschuk aufzufassen. Insbesondere haben sich Alginate und synthetische Quellstoffe, wie z.B. Natriumcarboxymethylcellulose, hochmolekulare Ethylenoxid-Copolymere, Salze der Poly(vinylether-co-maleinsäure) und Polyacrylamide.

Als Hilfsstoffe für pastöse und flüssige Produkte eignen sich besonders hydrophobe Grundlagen, insbesondere Kohlenwasserstoffe, wie beispielsweise Weißes Vaselin (DAB) oder Paraffinöl.

Die erfindungsgemäßen Verbindungen sind zur Hemmung von Mikrobenwachstum überall dort geeignet, wo dieses Wachstum unerwünscht ist, z. B. in wässrigen Systemen bei einer Reihe industrieller Anwendungen, wie der Papierherstellung.

Eine Reihe wichtiger Industrien wird durch die Aktivität dieser Bakterien, Algen und Pilze an den eingesetzten Rohmaterialien, an verschiedenen Aspekten ihrer Herstellungstätigkeiten oder an den hergestellten Endprodukten stark beeinträchtigt. Zu diesen Industrien gehören die Farben-, Holz-, Textil-, Kosmetik-, Leder-, Tabak-, Pelz-, Seil-, Papier-, Zellstoff-, Kunststoff-, Treibstoff-, Öl-, Kautschuk- und Maschinenindustrie.

Zu wichtigen Anwendungen der erfindungsgemäßen Verbindungen gehören: Hemmung des Wachstums von Bakterien und Pilzen in wässrigen Farben, Klebstoffen, Latexemulsionen und Vergussmassen; Holzkonservierung; Bohrölkonservierung; Bekämpfung schleimproduzierender Bakterien und Pilze in Zellstoff-und Papiermühlen und Kühlwasser; als Sprüh- oder Tauchbehandlung für Textilien und Leder zur Verhinderung von Schimmelpilzwachstum; als Komponente in Antifäulnisfarben zur Verhinderung der Anhaftung von Fäulnisorganismen; Schutz von Anstrichfilmen, insbesondere Außenfarben, vor dem Angriff durch Pilze, der bei der Verwitterung des Anstrichfilms auftritt; Schutz von Verarbeitungsausrüstung vor Schleimablagerungen bei der Herstellung von Rohr- und Rübenzucker; Verhinderung der Anreicherung und Ablagerung von Mikroorganismen in Abluftwäscher-Systemen und in industriellen Frischwasserversorgungssystemen; Bekämpfung der Kontamination mit und Ablagerung von Mikroorganismen in Ölbohrflüssigkeiten und -schlämmen sowie in sekundären Erdölaufbereitungsverfahren; Hemmung von Bakterien- und Pilzwachstum bei Papierbeschichtungsverfahren, das die Qualität der Papierbeschichtung beeinträchtigen könnte; Bekämpfung von Bakterien- und Pilzwachstum und -ablagerungen bei der Herstellung verschiedener Spezialpappen, z. B. Vollpappe und Spanplatten; Verhinderung der Zellsaftverfärbung von frisch geschlagenem Holz unterschiedlicher Art; Bekämpfung von Bakterien- und Pilzwachstum in Ton-und Pigmentschlämmen unterschiedlicher Art, die zur späteren Verwendung beispielsweise bei der Papierbeschichtung und der Farbenherstellung hergestellt werden und bei der Lagerung und beim Transport dem Abbau durch Mikroorganismen unterliegen; als Desinfektionsmittel für harte Oberflächen zur Verhinderung des Wachstums von Bakterien und Pilzen auf Wänden, Böden usw. sowie in Schwimmbecken zur Verhinderung von Algenwachstum.

Besonders wichtig ist die Bekämpfung von Bakterien und Pilzen in Wassersystemen von Zellstoff- und Papiermühlen, die wässrige Dispersionen der Fasern zur Papierherstellung enthalten. Die unkontrollierte Anreicherung von Schleim durch die Anhäufung von Bakterien und Pilzen führt zu qualitativ minderwertiger Produktion, verringerter Produktion aufgrund von Pausen und höherer Reinigungshäufigkeit, gesteigertem Verbrauch von Rohmaterialien sowie höheren Wartungskosten. Das Problem der Schleimablagerungen wird in der Papierindustrie durch die weitverbreitete Verwendung geschlossener Weißwassersysteme verschlimmert.

Ein weiterer wichtiger Bereich, in dem die Bekämpfung von Bakterien- und Pilzwachstum entscheidend ist, sind die Ton- und Pigmentschlämme. Diese Schlämme bestehen aus verschiedenen Tonen, z. B. Kaolin, und Pigmenten, z. B. Calciumcarbonat und Titandioxid. Sie werden gewöhnlich an einem Ort hergestellt, der von dem der endgültigen Verwendung, z. B. in der Papierbeschichtung und der Farbenherstellung, entfernt ist und dann zum späteren Transport an den Endverbrauchsort gelagert. Die hohen Qualitätsstandards für die Papier- und Farbenendprodukte, in denen der Schlamm verwendet wird, erfordern, dass der Ton- oder Pigmentschlamm einen sehr geringen Mikroorganismengehalt besitzt, damit er zur Papierbeschichtung oder Farbenherstellung eingesetzt werden kann.

Ein weiteres wichtiges Gebiet zur Bekämpfung von Mikrobenwachstum sind Kühlsysteme, wie diejenigen mit Umlaufkühltürmen. Diese Systeme setzen eine große Menge Wasser beträchtlich lange der Atmosphäre aus unter Bedingungen, die keine ausreichende Belüftung und kein ausreichendes Aussetzen gegenüber Sonnenlicht beinhalten, dass Mikrobenwachstum, insbesondere Bakterien- und Pilzwachstum bekämpft würde. Viele Kühltürme setzen ausserdem eine Füllung aus Kügelchen aus synthetischen Polymer- oder anderen Materialien ein, um die Wärmeaustauschoberfläche zu vergrößern. Diese Bauweise verschlimmert das Problem des Mikrobenwachstums, da sie die ideale physikalische Umgebung für die Vermehrung lästiger Mikroben bereitstellt. Unbekämpft gedeihen diese Mikroorganismen und erzeugen Kolonien, die ausreichen, dass die Wärmeaustauschoberflächen mit einem Biofilm blockiert und die Komponenten der Wassertransportvorrichtung, die zum Betrieb des Kühlsystems verwendet wird, verstopft werden. Die erfindungsgemäßen Verbindungen stellen eine ausgezeichnete Bekämpfung von Mikrobenwachstum in diesen Systemen bereit.

Die erfindungsgemäßen Verbindungen sind besonders geeignet zur Bekämpfung der schädlichen Wirkungen von Mikroorganismen in Wasser oder wässrigen Medien. Systeme, die umlaufendes Wasser oder umlaufende wässrige Medien verwenden, werden mit Mikroorganismen infiziert und erheblich in ihrer Wirksamkeit beeinträchtigt, wenn sich Mikroorganismenablagerungen im System anreichern. Die als Schleime bezeichneten Ablagerungen überziehen die Wände von Behältern und anderen Gefäßen, jegliche verwendete Maschinen und Verarbeitungsausrüstung und erzeugen Verstopfungen in Rohren und Ventilen. Die Schleimentstehung fördert die Korrosion von Metalloberflächen und erleichtert die Verrottung von Holztürmen. Die Schleime erzeugen auch Verfärbungen und andere Mängel in allen hergestellten Produkten und erzwingen kostenintensive Betriebsunterbrechungen. Die Bekämpfung von Mikroorganismen in wässrigen Medien ist besonders wichtig, wenn sich in diesen dispergierte Teilchen oder Feinstoffe befinden, z. B. dispergierte Zellulosefasern und dispergierte Füllstoffe und Pigmente bei der Papierherstellung sowie dispergierte Pigmente bei der Farbenherstellung.

Die erfindungsgemäßen Verbindungen können in reiner Form oder als wirksame Bestandteile von Gemischen eingesetzt werden, beispielsweise als Bestandteile von Sterilisations-, Desinfektions- Imprägnier- oder Konservierungsmitteln.

In der Mehrzahl der Fälle enthalten die für die praktische Anwendung bestimmten Gemische insgesamt noch 0 bis etwa 99, vorzugsweise 90 bis 10 Gew.-% weitere üblicherweise verwendete Bestandteile, die je nach der vorgesehenen Anwendungsform und dem Anwendungszweck ausgewählt werden.

Für flüssige Zubereitungen beispielsweise kommen als Lösungsmittel mit Wasser mischbare organische Lösungsmittel in Betracht, beispielsweise Ethanol, Isopropanol und Ethylenglykol, Propylenglykol, Ethylethylenglykol, Propylpropylenglykol 20 sowie mit Wasser nicht mischbare Lösungsmittel wie beispielsweise Testbenzin, Benzol, Toluol, Essigsäureethy-lester oder Dimethylenchlorid.

Wenn neben der keimhemmenden Wirkung eine zusätzliche Reinigungswirkung erwünscht ist, können die erfindungsgemäßen Gemische auch noch Tenside, insbesondere nichtionische Tenside enthalten. Beispiele für geeignete Tenside sind C₈-C₁₈-Alkylglucoside mit etwa 1 bis 10 Glucoseeinheiten im Molekül, Anlagerungs-Produkte von 4 bis 40, vorzugsweise 4 bis 20 Mol Ethylenoxid an ein Mol Fettalkohol, Alkylcyclohexanol, Alkylphenol, Fettsäure, Fettsäureamid oder Alkansulfonamid. Von besonderem Interesse sind Anlagerungsprodukte von 5 bis 16 Mol Ethylenoxid an Kokos- oder Talgfettalkohole, an Oleylalkohol, ein Gemisch aus Oleylalkohol und Cetylalkohol sowie an Mono-, Di- oder Trialkylphenole und an Monoalkylcyclohexanole mit 6 bis 14 Kohlenstoffatomen in den Alkylresten.

Auch gemischte Anlagerungsprodukte von Ethylenoxid und Propylenoxid an die genannten Verbindungen mit einem aktiven Wasserstoffatom kommen in Betracht.

Die genannten Alkoxylierungsprodukte können auch endgruppenverschlossen sein, beispielsweise durch Ether-oder Acetalgruppen.

In den erfindungsgemäßen Gemischen können ferner Gerüstsubstanzen vorhanden sein; als solche eignen sich beispielsweise Alkalisalze der Glukonsäure, insbesondere Natriumglukonat, die Alkalisalze der Nitrilotriessigsäure, Ethylendiamintetraessigsäure, Hydroxyethandiphosphonsäure, Phosphonobutantricarbonsäure, Milchsäure, Zitronensäure oder Weinsäure. Weiterhin kommen als Gerüstsubstanzen die wasserlöslichen Salze höhermolekularer Polycarbonsäuren in Betracht, etwa Polymerisate der Maleinsäure, Itakonsäure, Fumarsäure und Zitraconsäure. Auch Mischpolymerisate dieser Säuren untereinander oder mit anderen polymerisierbaren Monomeren, wie B. Ethylen, Propylen, Acrylsäure, Vinylacetat, Isobutylen, Acrylamid und Styrol sind brauchbar. In die erfindungsgemäßen Gemische können auch Reinigungsverstärker wie Fettsäuremono- und -diethanolamide, beispielsweise Kokosfettsäuremonoethanolamid und Kokosfettsäurediethanolamid, und Anlagerungsprodukte von bis zu 4 Mol Ethylenoxid oder Propylenoxid an Fettalkohole mit 8 bis 12 Kohlenstoffatomen sowie freie Fettalkohole mit 8 bis 12 Kohlenstoffatomen sowie Reinigungsverstärker auf Cellulosebasis eingearbeitet werden.

Darüber hinaus kann es für weitere Anwendungsbereiche vorteilhaft sein, wenn die Mittel zusätzlich weitere antimikrobiell wirksame Substanzen enthalten. In die erfindungsgemäßen Mittel können auch Insekticide wie z. B. Pyrethroide (Permethrin, Cypermethrin, Decamethrin und Fenvalerate) und/oder Lindan, Endosulfan, Dieldrin eingearbeitet werden.

Die Mengen der möglichen zur Konfektionierung der erfindungsgemäßen Mittel benutzten Bestandteile richten sich im allgemeinen nach Handels- und Preisvorgaben und sind im Prinzip nicht von erfinderischer Bedeutung.

Für die Herstellung gebrauchsfertiger Konservierungsmittel können neben flüssigen Konzentraten auch feste Produkte, vorzugsweise in Pulver- oder Granulatform bereitgestellt werden, die die erfindungsgemäßen Verbindungen gemäß Formel I enthalten.

Die erfindungsgemäßen Desinfektions- und Konservierungsmittel können auf vielen Gebieten zum Einsatz gelangen, beispielsweise in Haushalten und im Gewerbe wie z. B. Krankenhäusern, Schulen, Badeanstalten, öffentlichen Verkehrsmitteln, gewerblichen Betrieben und Industrieanlagen.

Weiterhin können die erfindungsgemäßen Verbindungen bei der Konservierung noch zu verarbeitender technischer Produkte wie Lasuren, Dispersions- und E-mulsionsfarben, Klebstoffen und Kleistern, Bohr- und Schneidölen oder Produkten der papier-, pappe- oder lederverarbeitenden Industrie sowie zur Konservierung von Industrie- und Brauchwasser Anwendung finden.

Die Applikation kann etwa durch Sprühen, Pinseln, Streichen, Rakeln, Tauchen oder Druck- oder Vakuumimprägnierung erfolgen

Die Verbindungen gemäß Formel I werden vorzugsweise in einer Konzentration im Bereich von 1 ppm bis 1000 ppm, insbesondere 20 bis 500 ppm, besonders bevorzugt 20 bis 100 ppm, insbesondere 20 bis 50 ppm, eingesetzt.

Die folgenden Beispiele erläutern die Erfindung, ohne sie jedoch darauf einzuschränken:

### Beispiele:

### a) Synthese von Alkyloxylactonen

### Beispiel 1: Synthese von α-Octyloxy-γ-Butyrolacton

| Ansatz: | | | |
|---|---|---|---|
| | 5,10 g | α-Hydroxy-γ-butyrolacton | 50 mmol |
| | 9,66 g | 1-Bromoctan | 50 mmol |
| | 16,29 g | Cäsiumcarbonat | 50 mmol |
| | 80 ml | Dimethylformamid (DMF) | |

### Durchführung:

50 mmol α-Hydroxy-y-butyrolacton wird in absolutem Dimethylformamid vorgelegt. Zu dieser Lösung werden 50 mmol Cäsiumcarbonat zugeben und innerhalb von 5 min 50 mmol Bromoctan (gelöst in absolutem Dimethylformamid) zugetropft. Das beschriebene Reaktionsgemisch wird für 76 h auf T = 70 °C erwärmt, danach abgekühlt und filtriert. Die Reaktionskontrolle erfolgte mittels Dünnschichtchromatographie und GLC. Nach dem Entfernen des Lösungsmittel bleibt ein rot-brauner Rückstand zurück, der fraktioniert destilliert werden kann.
Ausbeute 20,6 g (85%) einer klaren Flüssigkeit, Sdp. (0,08 mbar) 156-161 °C

### Beispiel 2: Synthese von α-Octyloxy-D-(-)-Pantolacton

| Ansatz: | | | |
|---|---|---|---|
| | 13,0 g | Pantolacton | 100 mmol |
| | 19,3 g | 1-Bromoctan | 100 mmol |
| | 32,6 g | Cäsiumcarbonat | 100 mmol |
| | 200 ml | Dimethylformamid (DMF) | |

### Durchführung:

100 mmol D-(-)-Pantolacton werden in absolutem Dimethylformamid vorlegt. Zu dieser Lösung werden 100 mmol Cäsiumcarbonat zugeben und innerhalb von 5 min 100 mmol Bromoctan (gelöst in absolutem Dimethylformamid) zugetropft. Das beschriebene Reaktionsgemisch wird für 48 h bei RT gerührt, danach filtriert und einrotiert. Die Reaktionskontrolle wurde mittels Dünnschichtchromatographie und GLC durchgeführt. Nach dem Entfernen des Lösungsmittel bleibt eine klare Flüssigkeit zurück, die im Hochvakuum fraktioniert destilliert werden kann. Ausbeute 5,06 g (48%) einer klaren Flüssigkeit (0,06 mbar) 127-150 °C

### Beispiel 3: Synthese von (R)-α-Octyloxy-γ-Butyrolacton

| Ansatz: | | | |
|---|---|---|---|
| | 2,00 g | R-(+)-α-Hydroxy-γ-butyrolacton | 19 mmol |
| | 3,67 g | 1-Bromoctan | 19 mmol |
| | 6,25 g | Cäsiumcarbonat | 19 mmol |
| | 130 ml | Dimethylformamid (DMF) | |

### Durchführung:

19 mmol R-(+)-α-Hydroxy-γ-butyrolacton werden in absolutem Dimethylformamid vorlegt. Zu dieser Lösung werden 19 mmol Cäsiumcarbonat zugeben und innerhalb von 5 min 19 mmol Bromoctan (gelöst in absolutem Dimethylformamid) zugetropft. Reaktionskontrolle erfolgt mittels GLC. Das beschriebene Reaktionsgemisch wird für 91 h auf T = 50°C erwärmt. Nach beendeter Reaktion wird das Reaktionsgemisch abgekühlt und filtriert. Nach Entfernen des Lösungsmittel wird das Rohpodukt mittels Flash-Säulenchromatographie gereinigt.
Ausbeute 2,34 g (58%), hellgelbe klare Flüssigkeit

### Beispiel 4: Synthese von α-Hexadecyloxy-γ-Butyrolacton

| Ansatz: | | | |
|---|---|---|---|
| | 5,10 g | α-Hydroxy-γ-butyrolacton | 50 mmol |
| | 15,74 g | 1-Bromhexadecan | 50 mmol |
| | 16,46 g | Cäsiumcarbonat | 50 mmol |
| | 80 ml | Dimethylformamid (DMF) | |

### Durchführung:

50 mmol α-Hydroxy-γ-butyrolacton werden in absolutem Dimethylformamid vorlegt. Zu dieser Lösung werden 50 mmol Cäsiumcarbonat zugeben und innerhalb von 5 min 50 mmol 1-Bromhexadecan (gelöst in absolutem Dimethylformamid) zugetropft. Das beschriebene Reaktionsgemisch wird für ca. 119 h auf T = 70 °C erwärmt und die Reaktion mittels GLC kontrolliert. Nach beendeter Reaktion wird das Reaktionsgemisch abgekühlt und filtriert. Nach Entfernen des Lösungsmittel wird das Rohpodukt mittels Flash-Säulenchromatographie gereinigt.
Ausbeute 5,53 g (34%) eines weißen Feststoffes

### b) Einsatz von Alkyloxylactonen zur Reduktion von Biofilmen auf Glasoberflächen

### Durchführung:

Der Keim *Pseudomonas aeruginosa* ATCC 15442 wird über Nacht auf Caso-Agar bei 37°C angezüchtet. Eine KBE (Kolonie-Bildende Einheit) vom Festmedium wird in 50 ml Caso-Bouillon für 7h bei 37°C und 150 upm inkubiert (1. Passage). Von der gewachsenen Flüssigkultur werden 100µl Keime in 50 ml frisches Caso-Nährmedium überführt und für ca. 16h bei 37°C und 150 upm bebrütet. (2. Passage). Aus dieser zweiten Passage wird im Biofilm-Test eine Keimzahl von 10⁶ KBE/ml eingesetzt. Für den Biofilm-Versuch werden die Keime in oben genannter Konzentration zusammen mit einem verdünnten Vollmedium (20fach mit DGHM-Wasser verdünntes TBY) in eine Mikrotiterplatte (6-well Kammer) pipettiert, welches als miniatu risiertes Biofilmtestsystem verwendet wird. Zu dem Gemisch (3ml) werden die zu testenden Wirkstoffe in der gewünschten Konzentration zugegeben. Als Kontrollen werden zum einen Ansätze mit Wirkstoff, aber ohne Keim eingesetzt, da die Wirkstoffe schon eine Eigenfärbung haben könnten (Negativkontrolle), zum anderen wird aber auch verdünntes Vollmedium mit Keim, aber ohne den Wirkstoff (Alkoxylacton) als Kontrolle eingesetzt. In die 6-well Kammern wird zusätzlich je 1 steriles Glasplättchen (Deckglas für Mikroskopie) der Größe 18x18 gelegt, auf dessen Oberfläche der Biofilmaufwuchs untersucht wird. Es werden von jedem Ansatz Dreifachbestimmungen durchgeführt, d.h. es werden drei Deckgläser pro Ansatz untersucht. Die 6-well Platten werden 6h bzw. 24h bei 30°C und 60 upm geschüttelt. Nach den vorgegebenen Inkubationszeiten werden pro Ansatz je 1ml zur Keimzahlbestimmung abgenommen, in Trypton-NaCl-Lösung verdünnt und auf Caso-Agar ausplattiert. Die resultierenden Platten werden 24h bei 37°C bebrütet und danach ausgezählt. Die Deckgläser werden zum Trocknen bei Raumtemperatur aus den 6-wells heraus genommen und anschließend in neuen 6-wells mit je 3ml 0,01 %ige SafraninO -Lösung (angesetzt mit sterilem Wasser) 15 Minuten angefärbt. Danach wird die Färbelösung abgesaugt, nicht-gebundener Farbstoff mit Wasser von den Deckgläsern entfernt und die gefärbten Deckgläser getrocknet. Die angefärbten und getrockneten Oberflächen der Deckgläser werden eingescannt und mit Corel Draw Paint 9 ausgewertet. Um den Hintergrundwert (verursacht durch das Glas) vom Messwert abziehen zu können, werden zusätzlich unbehandelte Deckgläser mitgescannt.

### Ergebnisse:

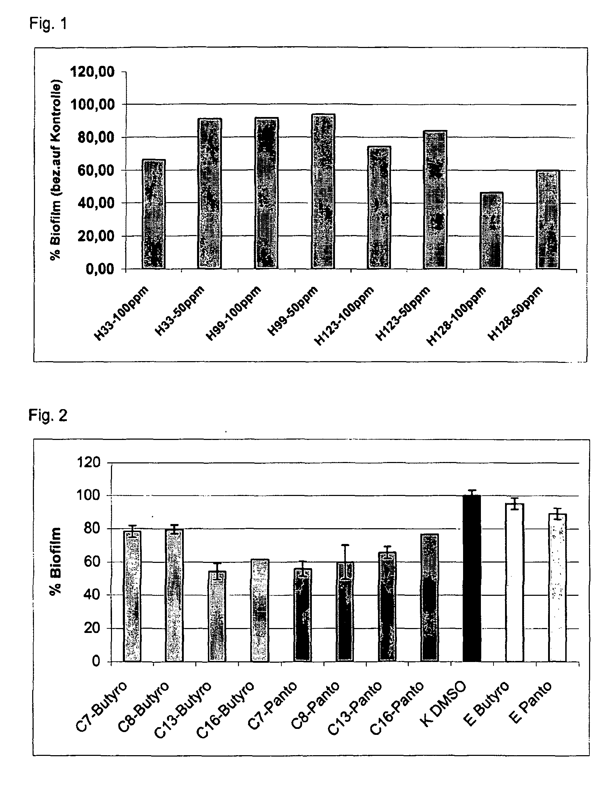

Die Ergebnisse sind in Fig. 1 dargestellt. In ersten orientierenden Versuchen (drei voneinander unabhängigen Versuche) waren nach 6 Stunden Inkubation Unterschiede erkennbar in der Biofilmbildung zur Kontrolle ohne Wirkstoffzusatz (in Fig.1 auf 100% gesetzt) im Vergleich zu den Ansätzen mit den Alkyloxylactonen. Es wurden jeweils 50ppm und 100ppm Wirkstoffe zu den Ansätzen hinzugefügt. Der Ansatz mit 100ppm Octyloxybutyrolacton (H128) zeigte eine Biofilmreduktion von ca 40% (bezogen auf die Kontrolle), bei Erhöhung der Wirkstoffkonzentration kam es zu Ausfällungen des Wirkstoffes und zu einer Reduzierung der Biofilm-Inhibierung auf 20% (Ergebnis nicht gezeigt).

Es besteht zumindest hier eine Abhängigkeit von der Konzentration. Neben dem Octyloxybutyrolacton (H128) zeigte Octyloxypantolacton (H33) den besten Effekt (über 30% Biofilm-Reduktion bei 100ppm). Die anderen Wirkstoffe (H99: Butyloxybutyrolacton; H123: Butyloxypantolacton) zeigten nur einen moderaten Effekt auf die Biofilm-Bildung. Das Zellwachstum wurde in allen Ansätzen nicht gestört.

Für weiterführende Versuche wurden neue, größere Substanzmengen synthetisiert sowie weitere Alkoxylacton-Derivate hergestellt (Tabelle 1; Fig. 2). Von allen Verbindungen wurde mittels Gaschromatographie der Lösungsmittelrestgehalt bestimmt, der in allen Fällen im unteren Nachweisbereich lag (Reinheit >97% laut GC-Untersuchungen), um sekundäre Einflüsse von Lösungsmitteln wie Toluol oder Dichlormethan auszuschließen.

Nach 6 Stunden Inkubation sind Unterschiede in der Biofilmbildung bei der DMSO-Kontrolle (in Fig. 2 auf 100% gesetzt) im Vergleich zu den Ansätzen mit verschiedenen Alkoxylactonen (Tab.1) zu erkennen. Es wurden jeweils 100ppm Wirkstoffe zu den Ansätzen hinzugegeben. Der Ansatz mit 100ppm Heptylpantolacton (188) zeigte eine Biofilmreduktion von über 40% (bezogen auf die Kontrolle). Die Pantolacton-Derivate mit einer längeren Seitenkette an R3 (Formel I) zeigten mit zunehmender Seitekette geringere Auswirkungen auf die Biofilm-Bildung (Fig. 2, dunkelgraue Balken). Bei den Butyrolacton-Derivaten (Fig. 2, dunkelgraue Balken) zeigte dagegen die Verbindung mit der längsten Seitenkette (Hexadecylbutyrolacton) die beste Biofilmreduktion, während die Derivate mit kurzer Seitenkette nur eine Biofilm-Verhinderung von 20% bewirkten. Ohne nennenswerte Wirkung waren die Edukte Hydroxybutyrolacton und Pantolacton (Fig. 2, schraffierte Balken). Das Zellwachstum wurde in allen untersuchten Ansätzen nicht gestört, d.h. es waren keine bioziden Effekte zu beobachten.

**Tabelle 1: Untersuchte Alkoxylacton-Derivate**

| | Heptyloxybutyrolacton |
|---|---|
| C8-Butyro | Octyloxybutyrolacton |
| C13-Butyro | Tridecyloxybutyrolacton |
| C16-Butyro | Hexadecyloxybutyrolacton |
| C7-Panto | Heptyloxypantolacton |
| C8-Panto | Octyloxypantolacton |
| C13-Panto | Tridecyloxypantolacton |
| C16-Panto | Hexadecyloxypantolacton |
| K DMSO | DMSO-Kontrolle |
| E Butyro | Edukt Hydroxybutyrolacton |
| IE Panto | Edukt Pantolacton |

### Abbildungen

Fig. 1: Auswirkungen von verschiedenen Alkoxylactonen auf die Biofilm-Bildung von *P. aeruginosa.* Die Angaben beziehen sich auf die Kontrolle ohne Wirksubstanz (= 100 %).
Fig. 2: Einfluss der Seitenketten von Alkoxylactonen auf die Biofilm-Bildung bei P. aeruginosa (schwarz: DMSO-Kontrolle; hellgrau: Butyrolacton-Derivate; dunkelgrau: Pantolacton-Derivate; schraffiert: Edukte Hydroxybutyrlacton und Pantolacton)

## Patentansprüche

1. Verbindung der Formel I, 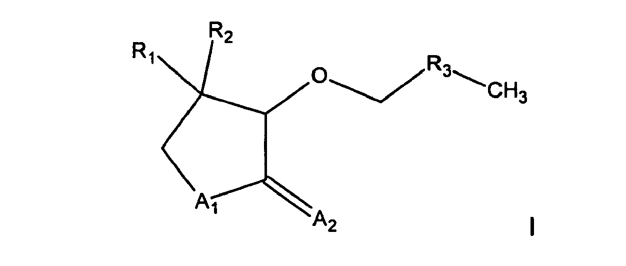
in der A₁ für O oder NH steht,
A₂ für O oder S steht,
R₁ und R₂ unabhängig voneinander für einen Rest stehen, der ausgewählt ist unter Wasserstoff, Methyl-, oder C₂-C₈-gesättigten oder ein- oder zweifach ungesättigten, verzweigten oder linearen Kohlenwasserstoffresten, und
R₃ für einen Rest steht, der ausgewählt ist unter C₃-C₁₈-gesättigten oder ein- oder zweifach ungesättigten, verzweigten oder linearen Kohlenwasserstoffresten,
wobei unabhängig voneinander R₁, R₂ und R₃ auch ein Heteroatom ausgewählt aus O und S in der Kette enthalten können und/oder ein- oder mehrfach substituiert sein können durch Reste ausgewählt aus Halogen, Hydroxy, C₁-C₆-Alkyl, Trifluormethyl, C₁-C₆-Alkoxy, C₆-C₁₀-Aryl, und C₁-C₆-Alkyl-C₆-C₁₀-aryl.

2. Verbindung nach Anspruch 1, in der A₁ und A₂ für O stehen, R₁ und R₂ für Wasserstoff oder Methyl stehen, und R₃ für Butylen, Pentylen, Hexylen, Heptylen, Octylen, Nonylen, Decylen, Undecylen, Dodecylen, Tridecylen oder Tetradecylen steht.

3. Verfahren zur Herstellung einer Verbindung nach Anspruch 1 oder 2, **dadurch gekennzeichnet, daß** man ein entsprechendes Lacton, Lactam oder Thiolactam mit einem 1-Halogenalkan in einem geeigneten organischen Lösungsmittel in Anwesenheit einer geeigneten Base umsetzt.

4. Verfahren zur Kontrolle von auf mikrobieller Interaktion beruhenden Vorgängen mit Ausnahme von Verfahren zur therapeutischen Behandlung des menschlichen oder tierischen Körpers, **dadurch gekennzeichnet, daß** man
a) gegebenenfalls die interagierenden Mikroorganismen bestimmt,
b) gegebenenfalls unter den Verbindungen gemäß Formel I die geeignete Verbindung oder die geeigneten Verbindungen auswählt, und
c) die Verbindung oder die Verbindungen gemäß Formel I in für die gewünschte Kontrolle ausreichender Menge dem Medium zusetzt, in dem die mikrobielle Interaktion stattfindet.

5. Verfahren nach Anspruch 4, **dadurch gekennzeichnet, daß** die Mikroorganismen ausgewählt sind unter Bakterien, Algen oder Pilzen, insbesondere unter Bakterien, vorzugsweise unter Gram-negativen Bakterien.

6. Verfahren nach Anspruch 4 oder 5, **dadurch gekennzeichnet, daß** die mikrobielle Interaktion ausgewählt ist unter der Ausbildung und/oder Reifung von Biofilmen, multizellulärem Schwärmverhalten, der konzertierten Ausbildung von Antibiotika-Resistenzen, der konzertierten Synthese von Antibiotika, der konzertierten Synthese von Pigmentstoffen, der konzertierten Produktion extrazellulärer Enzyme, insbesondere hydrolytischer Enzyme, sowie der konzertierten Produktion von Virulenzfaktoren, vorzugsweise der Ausbildung und/oder Reifung von Biofilmen.

7. Verwendung einer oder mehrerer Verbindungen der Formel I zur Kontrolle von auf mikrobieller Interaktion beruhenden Vorgängen, insbesondere zur Kontrolle der Ausbildung und/oder Reifung von Biofilmen, besonders bevorzugt von Biofilmen, an denen Gram-negative Bakterien beteiligt sind, mit Ausnahme von Verwendungen zur therapeutischen Behandlung des menschlichen oder tierischen Körpers.

8. Verwendung nach Anspruch 7, **dadurch gekennzeichnet, daß** die Verwendung in Sterilisations-, Desinfektions- Imprägnier- oder Konservierungsmitteln, Wasch- oder Reinigungsmitteln, oder in Kühl- oder Kühlschmiermitteln erfolgt.

9. Verwendung nach Anspruch 7 oder 8, **dadurch gekennzeichnet, daß** die Verwendung auf dem Gebiet der Wasserreinigung sowie der Arzneimittel,-Lebensmittel-, Brauerei, Medizintechnik-,Farben-, Holz-, Textil-, Kosmetik-, Leder-, Tabak-, Pelz-, Seil-, Papier-, Zellstoff-, Kunststoff-, Treibstoff-, Öl-, Kautschuk- oder Maschinenindustrie erfolgt.

10. Verwendung nach einem der Ansprüche 7 bis 9, **dadurch gekennzeichnet, daß** die Verwendung zur Biofilmkontrolle bei medizinischen Geräten, Instrumenten und Apparaturen, insbesondere bei Kathetern und Endoskopen erfolgt.

11. Verwendung nach einem der Ansprüche 7 bis 10, **dadurch gekennzeichnet, daß** die Verbindung oder die Verbindungen gemäß Formel I in einer Konzentration im Bereich von 1 ppm bis 1000 ppm, insbesondere 20 bis 500 ppm und besonders bevorzugt 20 bis 100 ppm eingesetzt werden.

12. Körperpflegemittel, Haarwaschmittel, Haarpflegemittel, Schaumbäder, Duschbäder, Cremes, Gele, Lotionen, alkoholische und wäßrig/alkoholische Lösungen, Emulsionen, Wachs/ Fett-Massen, Stiftpräparate, Puder oder Salben; Mund-, Zahn- oder Zahnprothesenpflegemittel, Kosmetika, Waschmittel, Reinigungsmittel, Nachspülmittel, Handwaschmittel, Handgeschirrspülmittel, Maschinengeschirrspülmittel, Desinfektionsmittel und Mittel zur Behandlung von Lebensmitteln, Arzneimitteln, Filtermedien, Textilien, Pelzen, Papier, Fellen oder Leder, enthaltend Verbindungen gemäß Formel I.

## Claims

1. A compound of Formula I, 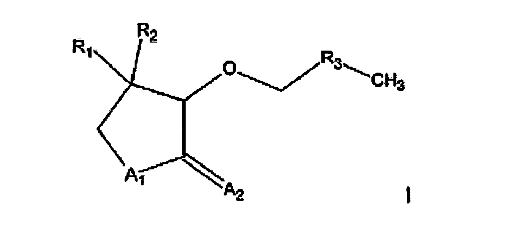 in which
A₁ stands for O or NH,
A₂ stands for O or S,
R₁ and R₂, independently of one another stand for a group selected from hydrogen, methyl or C₂-C₈ saturated or mono- or di-unsaturated, branched or linear hydrocarbon groups, and
R₃ stands for a group selected from C₃-C₁₈ saturated or mono- or di-unsaturated, branched or linear hydrocarbon groups,
wherein independently of one another R₁, R₂ and R₃ can also comprise a heteroatom selected from O and S in the chain and/or can be mono- or polysubstituted by groups selected from halogen, hydroxyl, C₁-C₆ alkyl, trifluoromethyl, C₁-C₆ alkoxy, C₆-C₁₀ aryl, and C₁-C₆ alkyl-C₆-C₁₀ aryl.

2. The compound according to claim 1, in which A₁ and A₂ stand for O, R₁ and R₂ stand for hydrogen or methyl, and R₃ stands for butylene, pentylene, hexylene, heptylene, octylene, nonylene, decylene, undecylene, dodecylene, tridecylene or tetradecylene.

3. Process for manufacturing a compound according to claim 1 or 2, **characterized in that** an corresponding lactone, lactam or thiolactam is treated with a 1-haloalkane in a suitable organic solvent in the presence of a suitable base.

4. Method for controlling processes based on microbial interaction with the exception of methods for the therapeutic treatment of the human or animal body, **characterized in that**
a) where necessary the interacting microorganisms are defined,
b) where necessary the appropriate compound or appropriate compounds are selected from the compounds according to Formula I, and
c) the compound or compounds according to Formula I are added to the medium, in which the microbial interaction takes place, in amounts sufficient for the desired control.

5. The method according to claim 4, **characterized in that** the microorganisms are selected from bacteria, algae or fungi, particularly from bacteria, preferably from Gram-negative bacteria.

6. The method according to claim 4 or 5, **characterized in that** the microbial interaction is selected from the development and/or maturation of biofilms, multicellular swarm behaviour, the concerted development of antibiotic resistances, the concerted synthesis of antibiotics, the concerted synthesis of pigment substances, the concerted production of extracellular enzymes, in particular hydrolytic enzymes, and the concerted production of virulence factors, preferably the development and/or maturation of biofilms.

7. Use of one or a plurality of compounds of Formula I for controlling processes based on microbial interaction, especially for controlling the development and/or maturation of biofilms, particularly preferably of biofilms in which Gram-negative bacteria are involved, with the exception of uses for the therapeutic treatment of the human or animal body.

8. Use according to claim 7, **characterized in that** the use is effected in sterilisation agents, disinfectants, impregnation agents or preservatives, detergents or cleansing agents, or in coolants or cooling lubricants.

9. Use according to claim 7 or 8, **characterized in that** the use is effected in the field of water purification, and the pharmaceutical, food, brewing, medical, colorant, wood, textile, cosmetic, leather, tobacco, hide, rope, paper, pulp, plastic, fuel, oil, rubber or machine industries.

10. Use according to one of claims 7 to 9, **characterized in that** the use is effected for biofilm control for medical equipment, instruments and apparatuses, particularly for catheters and endoscopes.

11. Use according to one of claims 7 to 10, **characterized in that** the compound or the compounds according to Formula I are employed in a concentration in the range 1 ppm to 1000 ppm, particularly 20 to 500 ppm and particularly preferably 20 to 100 ppm.

12. Body care agents, hair shampoos, hair care agents, bubble baths, shower baths, creams, gels, lotions, alcoholic and aqueous-alcoholic solutions, emulsions, wax/fatty masses, stick preparations, powders or salves, mouth-, tooth- or denture care products, cosmetics, cleansing agents, rinsing agents, hand detergents, hand dishwashing agents, automatic dishwasher agents, disinfectants and agents for treating foodstuffs, pharmaceuticals, filter media, textiles, hides, paper, skins or leather that comprise compounds according to Formula I.

## Revendications

1. Composé de formule I, 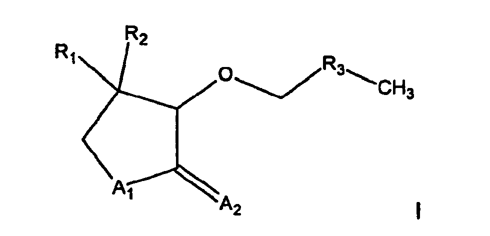 où
A₁ représente O ou NH,
A₂ représente O ou S,
R₁ et R₂ représentent, indépendamment l'un de l'autre, un radical choisi parmi l'hydrogène, le radical méthyle ou les radicaux hydrocarbonés en C₂-C₈, saturés ou monoinsaturés ou di-insaturés, ramifiés ou linéaires, et
R₃ représente un radical choisi parmi les radicaux hydrocarbonés en C₃-C₁₈, saturés ou monoinsaturés ou di-insaturés, ramifiés ou linéaires,
R₁, R₂ et R₃, indépendamment l'un de l'autre, pouvant également contenir un hétéroatome choisi parmi O et S dans la chaîne et/ou pouvant être monosubstitués ou polysubstitués par des radicaux choisis parmi halogène, hydroxy, C₁-C₆-alkyle, trifluorométhyle, C₁-C₆-alcoxy, C₆-C₁₀-aryle, et C₁-C₆-alkyl-C₆-C₁₀-aryle.

2. Composé selon la revendication 1, où A₁ et A₂ représentent O, R₁ et R₂ représentent hydrogène ou méthyle, et R₃ représente butylène, pentylène, hexylène, heptylène, octylène, nonylène, décylène, undécylène, dodécylène, tridécylène ou tétradécylène.

3. Procédé pour la préparation d'un composé selon la revendication 1 ou 2, **caractérisé en ce qu'**on transforme une lactone, un lactame ou un thiolactame correspondant avec un 1-halogénoalcane dans un solvant organique approprié en présence d'une base appropriée.

4. Procédé pour le contrôle de processus reposant sur une interaction microbienne à l'exception de procédés pour le traitement thérapeutique du corps humain ou animal, **caractérisé en ce qu'**on
a) détermine le cas échéant les microorganismes qui interagissent,
b) choisit le cas échéant parmi les composés selon la formule I le composé approprié ou les composés appropriés, et
c) ajoute le composé ou les composés selon la formule I en une quantité suffisante pour le contrôle souhaité au milieu dans lequel l'interaction microbienne a lieu.

5. Procédé selon la revendication 4, **caractérisé en ce que** les microorganismes sont choisis parmi les bactéries, les algues ou les champignons, en particulier parmi les bactéries, de préférence parmi les bactéries gram-négatives.

6. Procédé selon la revendication 4 ou 5, **caractérisé en ce que** l'interaction microbienne est choisie parmi la formation et/ou la maturation de biofilms, le comportement d'essaimage multicellulaire, la formation concertée de résistances aux antibiotiques, la synthèse concertée d'antibiotiques, la synthèse concertée de pigments, la production concertée d'enzymes extracellulaires, en particulier d'enzymes hydrolytiques, ainsi que la production concertée de facteurs de virulence, de préférence la formation et/ou la maturation de biofilms.

7. Utilisation d'un ou de plusieurs composés de formule I pour le contrôle de processus reposant sur une interaction microbienne, en particulier pour le contrôle de la formation et/ou de la maturation de biofilms, de manière particulièrement préférée de biofilms, auxquels participent des bactéries gram-négatives, à l'exception d'utilisations pour le traitement thérapeutique du corps humain ou animal.

8. Utilisation selon la revendication 7, **caractérisée en ce que** l'utilisation a lieu dans des agents de stérilisation, de désinfection, d'imprégnation ou de conservation, des agents de lavage ou de nettoyage ou dans des agents de refroidissement ou de lubrification à froid.

9. Utilisation selon la revendication 7 ou 8, **caractérisée en ce que** l'utilisation a lieu dans le domaine de l'épuration des eaux ainsi que de l'industrie des médicaments, des aliments, de brasserie, de technique médicale, des peintures, du bois, du textile, des cosmétiques, du cuir, du tabac, des fourrures, des cordes, du papier, de la cellulose, des matériaux synthétiques, des carburants, des huiles, du caoutchouc ou des machines.

10. Utilisation selon l'une quelconque des revendications 7 à 9, **caractérisée en ce que** l'utilisation pour le contrôle de biofilms a lieu sur des dispositifs, des instruments et des appareillages médicaux, en particulier sur des cathéters et des endoscopes.

11. Utilisation selon l'une quelconque des revendications 7 à 10, **caractérisée en ce que** le composé ou les composés selon la formule I sont utilisés en une concentration dans la plage de 1 ppm à 1000 ppm, en particulier de 20 à 500 ppm et de manière particulièrement préférée de 20 à 100 ppm.

12. Agents de soin corporel, agents de lavage des cheveux, agents de soin capillaire, bains mousse, bains douche, crèmes, gels, lotions, solutions alcooliques et aqueuses/alcooliques, émulsions, masses de cire/graisse, préparations en bâton, poudres ou onguents ; agents de soin buccaux, dentaires ou de prothèse dentaire, cosmétiques, agents de lavage, agents de nettoyage, agents de rinçage, agents de lavage à la main, détergents pour vaisselle à la main, détergents pour lave-vaisselle, désinfectants et agents pour le traitement d'aliments, de médicaments, de moyens de filtration, de textiles, de fourrures, de papiers, de peaux ou de cuir, contenant des composés selon la formule 1.
